# EUROPEAN PATENT SPECIFICATION

(11) **EP 1 686 190 B1**
(45) Date of publication and mention of the grant of the patent: **23.04.2008**
(21) Application number: 06001513.8
(22) Date of filing: 25.01.2006
(51) Int. Cl.: C12Q 1/68

(54) **Methods of genotyping using differences in melting temperature**
Verfahren zur Genotypisierung durch Schmelztemperaturabhängigkeit
Procédé de génotypage utilisant des différences en point de dénaturation

(30) Priority: 28.01.2005 US 647948 P; 09.03.2005 US 660187 P
(43) Date of publication of application: 02.08.2006
(73) Proprietor: Roche Diagnostics GmbH, 68305 Mannheim (DE); F.HOFFMANN-LA ROCHE AG, 4070 Basel (CH)
(72) Inventor: Germer, Soren, Maplewood, NJ 07040 (US); Higuchi, Russell Gene, Alameda, CA 94501 (US); Mirel, Daniel B., Cambridge, MA 02139 (US); Wang, Jun, Fremont, CA 94555 (US)

(56) References cited:
- LIU QIANG ET AL: "Overlapping PCR for bidirectional PCR amplification of specific alleles: A rapid one-tube method for simultaneously differentiating homozygotes and heterozygotes" PCR METHODS AND APPLICATIONS, COLD SPRING HARBOR, NY, US, vol. 7, no. 4, 1997, pages 389-398, XP002259043 ISSN: 1054-9803
- GERMER S ET AL: "SINGLE-TUBE GENOTYPING WITHOUT OLIGONUCLEOTIDE PROBES" GENOME RESEARCH, COLD SPRING HARBOR LABORATORY PRESS, WOODBURY, NY, US, vol. 9, no. 1, January 1999 (1999-01), pages 72-78, XP000851846 ISSN: 1088-9051
- DONOHOE G G ET AL: "RAPID SINGLE-TUBE SCREENING OF THE C282Y HEMOCHROMATOSIS MUTATION BY REAL-TIME MULTIPLEX ALLELE-SPECIFIC PCR WITHOUT FLUORESCENT PROBES" CLINICAL CHEMISTRY, AMERICAN ASSOCIATION FOR CLINICAL CHEMISTRY, WASHINGTON, DC, US, vol. 46, no. 10, 2000, pages 1540-1547, XP002906811 ISSN: 0009-9147
- PAPP AUDREY C ET AL: "Single nucleotide polymorphism genotyping using allele-specific PCR and fluorescence melting curves." BIOTECHNIQUES, vol. 34, no. 5, May 2003 (2003-05), pages 1068-1072, XP001208020 ISSN: 0736-6205
- BREEN G: "NOVEL AND ALTERNATE SNP AND GENETIC TECHNOLOGIES" PSYCHIATRIC GENETICS, RAPID COMMUNICATIONS OF OXFORD LTD, vol. 12, no. 2, June 2002 (2002-06), pages 83-88, XP009001223 ISSN: 0955-8829
- WANG JUN ET AL: "High-throughput SNP genotyping by single-tube PCR with T-m-shift primers" BIOTECHNIQUES, vol. 39, no. 6, December 2005 (2005-12), pages 885-893, XP001208019 ISSN: 0736-6205

## Description

### FIELD OF THE INVENTION

The present invention is related to the field of nucleic acid amplification.

### BACKGROUND OF THE INVENTION

The identification of complex biological traits that are affected by multiple genetic and environmental factors is a very difficult and challenging task. In contrast with monogenic traits, it is impossible to follow all of the genomic regions that are responsible for the complex variation of the trait without some further idea of how these regions segregate. A key development in the analysis of complex traits was the establishment of large collections of molecular/genetic markers including single nucleotide polymorphisms (SNPs) and other nucleotide polymorphisms, including base deletions, insertions, and multiple base changes. Several databases of nucleotide polymorphisms have been established and are steadily growing in content. Inexpensive and high-throughput genotyping technologies are needed to fully exploit the opportunity offered by nucleotide polymorphisms to detect allelic variation in genes involved in complex traits.

Many genotyping technologies have been developed in the last few years based on various methods of allele discrimination, target amplification and detection platforms. The detection mechanisms roughly fall into two categories: solid-phase-mediated detection and homogeneous detection. The solid-phase-mediated detection uses solid support in the detection step, which includes mass spectrometry, microarrays, microbeads and electrophoresis. Homogenous detection methods are done in solution from beginning to end and no separation or purification steps are needed. Many of the homogenous detection systems utilize fluorescent detection methods and one specific forward primer extended at the 5' end (e.g., Germer, S. and Higuchi R., 1999, Single-tube genotyping without oligonucleotide probes, Genome Research, Cold Spring Harbor Lab. Press, Woodbury, NY, US, vol. 9, no. 1, 72-78; Donohoe, G,G., Laaksonen, M., Pulkki, K., Rönnemaa, T. and Kairisto, V., 2000, Clin. Chem., vol. 45, no. 10, 1540-1547; Papp, A.C., Pinsonneault, J.K., Cooke, G. and Sadée, W., 2003, BioTechniques, vol. 34, no. 5, 1068-1072). Methods for performing allele-specific PCR can be also categorized as homogeneous detection methods.

Single-tube genotyping methods have been developed, but most require fluorescent-modified oligonucleotides, and the cost of these materials can be high. An inexpensive and robust homogenous genotyping method without using fluorescent probes is desirable.

### SUMMARY OF THE INVENTION

The present invention includes methods to determine a base at a particular nucleotide polymorphism site in a nucleic acid sample comprising the steps of: (a) amplifying the nucleic acid sample in an amplification reaction comprising a first forward primer comprising a 3' terminal base complementary to a first base at the particular nucleotide polymorphism site, and a first 5' non-complementary sequence, a second forward primer comprising a 3' terminal base complementary to a second base at the particular nucleotide polymorphism site, and a second 5' non-complementary sequence that is different in melting temperature than the first 5' non-complementary sequence, a reverse primer complementary to sequence downstream of the particular nucleotide polymorphism site, and a DNA polymerase; (b) creating an amplification reaction product; (c) measuring the melting temperature of the amplification reaction product; and (d) determining the base at the particular nucleotide polymorphism site by the melting temperature of the amplification reaction product.

In certain embodiments, the above described method may further include a third forward primer comprising a 3' terminal base complementary to a third base at the particular nucleotide polymorphism site, and a third 5' non-complementary sequence that is different in melting temperature than the first and second 5' non-complementary sequences. The invention may additionally include a fourth forward primer comprising a 3' terminal base complementary to a fourth base at the particular nucleotide polymorphism site, and a fourth 5' non-complementary sequence that is different in melting temperature than the first and second and third 5' non-complementary sequences.

In certain embodiments, a method is provided to determine a base at a particular nucleotide polymorphism site in a nucleic acid sample comprising the steps of: (a) amplifying the nucleic acid sample in an amplification reaction comprising a first forward primer comprising a penultimate 3' base complementary to a first base at the particular nucleotide polymorphism site and a first 5' non-complementary sequence, a second forward primer comprising a penultimate 3' base complementary to a second base at the particular nucleotide polymorphism site and a second 5' non-complementary sequence that is different in melting temperature than the first 5' non-complementary sequence, a reverse primer complementary to sequence downstream of the particular nucleotide polymorphism site, and a DNA polymerase; (b) creating an amplification reaction product; (c) measuring the melting temperature of the amplification reaction product; and (d) determining the base at the particular nucleotide polymorphism site by the melting temperature of the amplification reaction product.

Additionally, the above described method may include a third forward primer comprising a penultimate 3' base complementary to a third base at the particular nucleotide polymorphism site, and a third 5' non-complementary sequence that is different in melting temperature than the first and second 5' non-complementary sequences. And the method may further comprise a fourth forward primer comprising a penultimate 3' base complementary to a fourth base at the particular nucleotide polymorphism site, and a fourth 5' non-complementary sequence that is different in melting temperature than the first and second and third 5' non-complementary sequences.

In certain embodiments, a method is provided to determine two bases at a particular nucleotide polymorphism site in a nucleic acid sample comprising the steps of: (a) amplifying the nucleic acid sample in an amplification reaction comprising: a first forward primer comprising a first polymorphism at the 3' end of the first forward primer at the particular nucleotide polymorphism site, wherein the first polymorphism comprises two bases, and a first 5' non-complementary sequence, a second forward primer comprising a second polymorphism at the 3' end of the second forward primer at the particular nucleotide polymorphism site, wherein the second polymorphism comprises two bases that are different by at least one base than the first polymorphism, and a second 5' non-complementary sequence that is different in melting temperature than the first 5' non-complementary sequence, a reverse primer complementary to sequence downstream of the particular nucleotide polymorphism site, and a DNA polymerase; (b) creating an amplification reaction product; (c) measuring the melting temperature of the amplification reaction product; and (d) determining the two bases at the particular nucleotide polymorphism site by the melting temperature of the amplification reaction product.

Additionally the above described method may include one or more additional forward primers, each additional forward primer comprising an additional polymorphism at the 3' end of each additional forward primer at the particular nucleotide polymorphism site, wherein the additional polymorphism comprises two bases that differ by at least one base from each polymorphism on each forward primer at the particular nucleotide polymorphism site, and a 5' non-complementary sequence that is different in melting temperature than each 5' non-complementary sequence on each forward primer.

In certain embodiments, a method is provided to determine three bases at a particular nucleotide polymorphism site in a nucleic acid sample comprising the steps of: (a) amplifying the nucleic acid sample in an amplification reaction comprising: a first forward primer comprising a first polymorphism at the 3' end of the first forward primer at the particular nucleotide polymorphism site, wherein the first polymorphism comprises three bases, and a first 5' non-complementary sequence, a second forward primer comprising a second polymorphism at the 3' end of the second forward primer at the particular nucleotide polymorphism site, wherein the second polymorphism comprises three bases that are different by at least one base than the first polymorphism, and a second 5' non-complementary sequence that is different in melting temperature than the first 5' non-complementary sequence, a reverse primer complementary to sequence downstream of the particular nucleotide polymorphism site, and a DNA polymerase; (b) creating an amplification reaction product; (c) measuring the melting temperature of the amplification reaction product; and (d) determining the three bases at the particular nucleotide polymorphism site by the melting temperature of the amplification reaction product.

The above described method may further include one or more additional forward primers, each additional forward primer comprising an additional polymorphism at the 3' end of each additional forward primer at the particular nucleotide polymorphism site, wherein the additional polymorphism comprises three bases that differ by at least one base from each polymorphism on each forward primer at the particular nucleotide polymorphism site, and a 5' non-complementary sequence that is different in melting temperature than each 5' non-complementary sequence on each forward primer.

All of the above-described methods may further include that amplifying the nucleic acid sample, creating the amplification reaction product, and measuring the melting temperature may be carried out in a single tube. The methods may further comprise determining at least one second base at a second particular nucleotide polymorphism site in the nucleic acid sample in the one amplification reaction, also described as multiplex detection of multiple polymorphism sites in one reaction. The amplification reaction may be the Polymerase Chain Reaction (PCR) or other primer-mediated amplification methodologies. The DNA polymerase may lack 5'-3' exonuclease activity, may be modified to have hot start activity, and may be Taq Stoffel Fragment. The amplification reaction may contain a fluorescent double-stranded nucleic acid binding dye such as SYBR green, or an intercalating dye such as ethidium bromide. The hot start activity may result from the use of other moieties, including for example the use of an aptamer, or an anti-Taq antibody.

The present invention also describes kits comprising: a first forward primer comprising a 3' terminal base complementary to a first base at a particular nucleotide polymorphism site, and a first 5' non-complementary sequence, and a second forward primer comprising a 3' terminal base complementary to a second base at the particular nucleotide polymorphism site, and a second 5' non-complementary sequence that is different in melting temperature than the first 5' non-complementary sequence.

The above described kit may comprise a third forward primer comprising a 3' terminal base complementary to a third base at the particular nucleotide polymorphism site, and a third 5' non-complementary sequence that is different in melting temperature than the first and second 5' non-complementary sequences. And the kit may further comprise a fourth forward primer comprising a 3' terminal base complementary to a fourth base at the particular nucleotide polymorphism site, and a fourth 5' non-complementary sequence that is different in melting temperature than the first and second and third 5' non-complementary sequences.

In certain embodiments, a kit is described comprising: a first forward primer comprising a penultimate 3' base complementary to a first base at a particular nucleotide polymorphism site, and a first 5' non-complementary sequence, and a second forward primer comprising a penultimate 3' base complementary to a second base at the particular nucleotide polymorphism site, and a second 5' non-complementary sequence that is different in melting temperature than the first 5' non-complementary sequence.

The above described kit may further comprise a third forward primer comprising a penultimate 3' base complementary to a third base at the particular nucleotide polymorphism site, and a third 5' non-complementary sequence that is different in melting temperature than the first and second 5' non-complementary sequences. And the kit may further comprise a fourth forward primer comprising a penultimate 3' base complementary to a fourth base at the particular nucleotide polymorphism site, and a fourth 5' non-complementary sequence that is different in melting temperature than the first and second and third 5' non-complementary sequences.

In certain embodiments, a kit is provided comprising: a first forward primer comprising a first polymorphism at the 3' end of the first forward primer at the particular nucleotide polymorphism site, wherein the first polymorphism comprises two bases, and a first 5' non-complementary sequence, and a second forward primer comprising a second polymorphism at the 3' end of the second forward primer at the particular nucleotide polymorphism site, wherein the second polymorphism comprises two bases that are different by at least one base than the first polymorphism, and a second 5' non-complementary sequence that is different in melting temperature than the first 5' non-complementary sequence.

The above described kit may further comprise one or more additional forward primers, each additional forward primer comprising: an additional polymorphism at the 3' end of each additional forward primer at the particular nucleotide polymorphism site, wherein the additional polymorphism comprises two bases that differ by at least one base from each polymorphism on each forward primer at the particular nucleotide polymorphism site, and a 5' non-complementary sequence that is different in melting temperature than each 5' non-complementary sequence on each forward primer.

In certain embodiments, a kit is provided comprising: a first forward primer comprising a first polymorphism at the 3' end of the first forward primer at the particular nucleotide polymorphism site, wherein the first polymorphism comprises three bases, and a first 5' non-complementary sequence, and a second forward primer comprising a second polymorphism at the 3' end of the second forward primer at the particular nucleotide polymorphism site, wherein the second polymorphism comprises three bases that are different by at least one base than the first polymorphism, and a second 5' non-complementary sequence that is different in melting temperature than the first 5' non-complementary sequence.

The above described kit may further comprise one or more additional forward primers, each additional forward primer comprising an additional polymorphism at the 3' end of each forward primer at the particular nucleotide polymorphism site, wherein the additional polymorphism comprises three bases that differ by at least one base from each polymorphism on each forward primer at the particular nucleotide polymorphism site, and a 5' non-complementary sequence that is different in melting temperature than each 5' non-complementary sequence on each forward primer.

All of the above described kits may further comprise a reverse primer complementary to sequence downstream of the particular nucleotide polymorphism site. And the kits may further comprise a DNA polymerase, which may lack 5'-3' exonuclease activity, may have hot start activity, and may be Taq Stoffel Fragment. The hot start activity may result from other moieties, including for example the use of an aptamer, or an anti-Taq antibody.

All of the above described methods and kits may additionally include that the 5' non-complementary sequences may be comprised of any nucleotides, or at least 75% G and C nucleotides, up to 90% G and C nucleotides, or up to and including 100% G and C nucleotides. Additionally the non complementary sequences may comprise at least one of (a) nucleotide base analogs, and (b) chemically-modified nucleotide bases. The length of the non-complementary sequences may be from 2 base pairs to 25 base pairs, but may also extend to 40 base pairs or 50 base pairs. The first sequence may be 6 base pairs and may be of the sequence: 5'-GCGGGC-3' (SEQ ID NO-01), and the second sequence may be 14 base pairs and may be of the sequence: 5'-GCGGGCAGGGCGGC-3' (SEQ ID NO-02). Alternatively, the first sequence may be 3 base pairs and may be of the sequence: 5'-GCG-3' (SEQ ID NO-03).

In addition to the above described methods and kits for determining a base at a particular nucleotide polymorphism site by measuring the melting temperature of the amplification products, other methods of detection including but not limited to the use of capillary electrophoresis detection could be utilized in connection with, in addition to, or in place of the described methods. These other methods of detection could utilize the inherent features that the amplification products created by the above described methods could be separated by size, molecular weight, as well as by melting temperature. For example, in certain embodiments, a method is provided to determine a base at a particular nucleotide polymorphism site in a nucleic acid sample, comprising amplifying the nucleic acid sample in an amplification reaction comprising a first forward primer comprising a 3' terminal base complementary to a first base at the particular nucleotide polymorphism site and a first 5' non-complementary sequence, a second forward primer comprising a 3' terminal base complementary to a second base at the particular nucleotide polymorphism site and a second 5' non-complementary sequence that is different in melting temperature than the first 5' non-complementary sequence, a reverse primer complementary to sequence downstream of the particular nucleotide polymorphism site, and a DNA polymerase; creating an amplification reaction product; and determining the base at the particular nucleotide polymorphism site by detecting if the amplification reaction product is created from the first forward primer or the second forward primer.

### DETAILED DESCRIPTION OF THE FIGURES

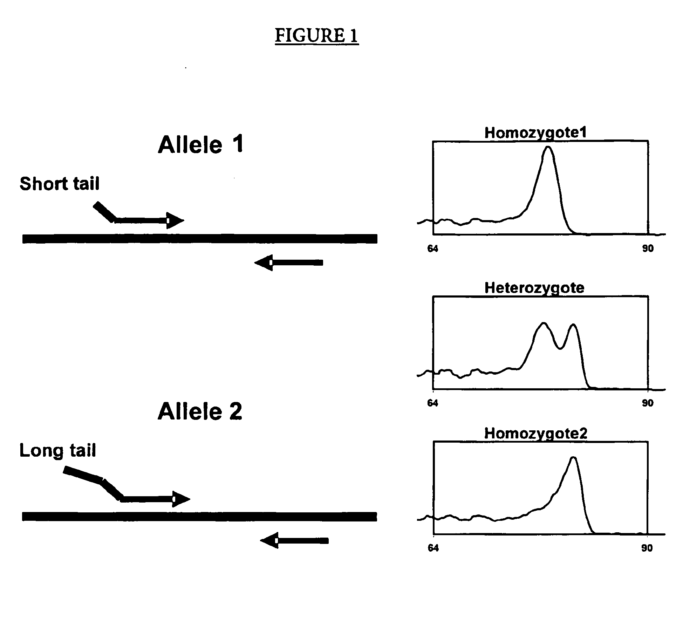

Figure 1 illustrates the principles of genotyping using differences in melting temperature. Nucleic acid is amplified with two forward allele-specific primers and a common reverse primer. A shorter non-complementary sequence "tail", comprising for example 6 base pairs or 3 base pairs, is attached to one allele-specific primer (specific to Allele 1) and a longer non-complementary sequence "tail", comprising for example 14 base pairs, is attached to the other allele-specific primer (specific to Allele 2). Samples homozygous for allele 1 are amplified only by the shorter primer and give a low temperature peak in a melting curve. Samples homozygous for allele 2 are amplified only by the longer primer and give a high temperature peak. The heterozygous samples are amplified by both primers and the melting curves result in two peaks.

### DETAILED DESCRIPTION OF THE INVENTION

### I. DEFINITIONS

Before describing the present invention in detail, it is to be understood that this invention is not limited to particular methods, compositions, reaction mixtures, kits, systems, computers, or computer readable media, which can, of course, vary. It is also to be understood that the terminology used herein is for the purpose of describing particular embodiments only, and is not intended to be limiting. Further, unless defined otherwise, all technical and scientific terms used herein have the same meaning as commonly understood by one of ordinary skill in the art to which this invention pertains. In describing and claiming the present invention, the following terminology and grammatical variants thereof will be used in accordance with the definitions set forth below.

"Allele-specific" refers to a reaction that is specific to one allele, where the reaction proceeds only if that specific allele is present. The term also refers to an oligonucleotide sequence being "allele-specific", where the oligonucleotide is matched to the sequence of a particular allele at a particular polymorphism site, and the oligonucleotide sequence is not matched to an alternative allele sequence at that same polymorphism site. Typically an allele-specific oligonucleotide acting as a primer will encompass the particular allele sequence at or near the 3' end of the primer.

"Nucleotide polymorphism" refers to the occurrence of two more alternative bases at a defined location that may or may not affect the coding sequence, gene or resulting proteins. The base changes may be a single base change, also known as a "single nucleotide polymorphism" or "SNP" or "snip". The base changes may be multiple base substitutions of the sequence at the location, and may include insertion and deletion sequence.

"Base" refers to the individual nucleotide components of an oligonucleotide, including a naturally occurring nucleotide or a nucleotide analog.

"Terminal base" refers to the last nucleotide base, or base analog, at the end of an oligonucleotide. The 3' terminal base is found at the 3' end of the oligonucleotide. The 5' terminal base is found at the 5' end of the oligonucleotide.

"Chemically-modified nucleotide base" refers to a nucleotide that has been modified to contain non-natural constituents.

"Nucleotide base analog" refers to a non-naturally occurring nucleotide base that may function as a naturally occurring nucleotide base.

"Penultimate 3' base" refers to the next-to-last nucleotide base, or base analog, at the 3' end of an oligonucleotide.

"Primer" refers to an oligonucleotide. An oligonucleotide used in PCR may function as a primer when it initiates the synthesis of a new DNA strand. An oligonucleotide used in PCR may also function as a "probe". Typically in PCR the oligonucleotide primer designed on the upper or coding strand of DNA (when the DNA sequence is written in "standard" left to right format) is known as the "forward" or "upstream" primer, and the oligonucleotide primer designed on the lower or noncoding strand of DNA is known as the "reverse" or "downstream" primer. However the "forward" and "reverse" primers could be designed on either strand of DNA.

"5' Non-complementary sequence" refers to a segment of sequence at or near the 5' end of an oligonucleotide that does not match the sequence found in the target DNA that is matched to the remainder of the oligonucleotide. This non-complementary sequence can comprise any naturally occurring nucleotide base or nucleotide base analog.

"Melting temperature" refers to the temperature at which 50% of the double stranded DNA molecule separates into two single stands of DNA. This temperature can be estimated by a number of methods, for example by a nearest-neighbor calculation as per Wetmur 1991 (Wetmur, J.G. 1991. DNA probes: applications of the principles of nucleic acid hybridization. Crit Rev Biochem Mol Biol 26: 227-259.

"Fluorescent double-stranded nucleotide binding dye" refers to a fluorescent dye compound that binds to double stranded nucleic acid. For example, this may include SYBR-Green I (Molecular Probes cat no. S7585).

"Fluorescent double-stranded nucleotide intercalating dye" refers to a fluorescent dye compound that intercalates into the double stranded nucleic acid. For example, this may include Ethidium Bromide.

"DNA polymerase" refers to an enzyme that synthesizes a new strand of DNA from a single stranded nucleic acid template. For example, this may include *Thermus aquaticus (Taq)* DNA polymerase I or derivatives thereof such as Stoffel fragment.

"Stoffel fragment" refers to a truncated form of *Taq* DNA polymerase I that does not contain 5'-3' exonuclease activity. (Applied Biosystems Part No. N808-0038; see also: Lawyer FC, Stoffel S, Saiki RK, Chang SY, Landre PA, Abramson RD, and Gelfand DH. 1993 high-level expression, purification, and enzymatic characterization of full-length *Thermus aquaticus* DNA polymerase and a truncated form deficient in 5' to 3' exonuclease activity. PCR Methods and Application 2:275-287.

**"5'-3' exonuclease minus"** refers to a polymerase that lacks 5'-3' exonuclease activity.

"**Hot start**" refers to the technique of delaying activity of a polymerase in an amplification reaction until after the initial heating steps above the temperature where the polymerase is active and at or near the temperature which melts the double-stranded DNA to single-stranded DNA. Examples of hot start techniques can include but are not limited to the use of a modified polymerase such as the "Gold" modification, the use of an aptamer, and the use of an anti-Taq antibody.

**"Gold"** refers to the modification of a polymerase as per the methods described in US patents US 5,677,152 and US 5,773,258.

"**Aptamer**" refers to a single-stranded DNA that recognizes and binds to DNA polymerase, and efficiently inhibits the polymerase activity as described in US 5,693,502.

"**Anti-Taq antibody**" refers to a polymerase inhibitor as described in US 6,140,086.

**"Multiplex"** refers to amplification with more than one set of primers, or the amplification of more that one polymorphism site in a single reaction.

### II. METHODS OF GENOTYPING USING DIFFERENCES IN MELTING TEMPERATURE

The present application describes several methods of genotyping, enabling a low-cost assay without fluorescent oligonucleotides and enabling a high success rate for the development of the assays. One feature of the present invention is the attachment of different 5' non-complementary sequences to each of the allele-specific primers, respectively. The resulting amplification products will have different dissociation properties due to the different non-complementary sequences. Amplification products from all alleles carry the non-complementary sequences, which reduces the imbalanced amplification reaction and dissociation characteristics of previously described methods. Samples that are homozygous and heterozygous at nucleotide polymorphism sites are distinguishable by these methods.

Allele-specific primers can be designed to amplify single nucleotide polymorphisms or multiple nucleotide polymorphisms or alleles, including substitution, insertion and deletion polymorphisms. One feature is that each allele-specific primer will match perfectly to the polymorphism sequence of interest to which it was designed and initiate the amplification reaction, and that the primer or primers that do not match perfectly to the polymorphism sequence of interest will not initiate the amplification reaction. A single nucleotide polymorphism site of interest may be interrogated by the final base at the 3' end of the allele-specific primer. Additionally, a single nucleotide polymorphism site of interest may be interrogated by the penultimate base at the 3' end of the primer. If the nucleotide polymorphism site of interest is two bases in length, then the site may be interrogated by the final two bases at the 3' end of the allele-specific primer. If the nucleotide polymorphism site consists of greater than 2 bases, then those nucleotides may be interrogated by a 2 to 6 bases pair stretch of sequence at the 3' end of the allele-specific primer. It is possible that allele-specific primers can be designed to interrogate greater than 6 bases at the 3' end of the primer.

At each nucleotide polymorphism site, or allele-site, multiple options for allele-specific primer 3' sequences may be found. For example, at a particular polymorphism site typically in a sample may be found a "G" base, but in certain samples may be found a "C" or and "A" base. In this example three unique primers would be designed, each differing at the 3' end of the primer, or differing at the penultimate 3' end of the primer, and comprising the base of interest. In a second example, at a particular polymorphism site typically in a sample may be found the sequence "CT", but in certain samples may be found the sequence "AG", "AT", or "CA". In this second example four unique primers would be designed, each differing at the last two bases of the 3' end of the primer and comprising the bases of interest. In general, the number of allele-specific primers possible at each polymorphism site (based on the four naturally occurring bases A, C, G and T) can be calculated based on the formula:
X = 4^{N}, where X equals the number of possible unique primers, and N equals the number of bases that differ at the polymorphism site. For example, at a certain site 3 bases can be polymorphic, such as "ACT" and "GTA". In this example, if one wishes to design all possible primer sequences, X = 4³ = 64 possible unique sequences.

One feature in the design of all of the allele-specific primers is the incorporation of unique sequences at each 5' end. Each allele-specific primer comprises a unique 5' end of non-complementary sequence (sequence that does not match the sequence upstream from the nucleotide polymorphism site). This unique 5' non-complementary sequence typically contains primarily G and C bases, but can also contain A and T bases. The sequence can be 90% G-C bases or greater up to and including 100% G-C bases, or at least 75% G-C bases. It is also possible that the 5' non-complementary sequence may contain non-naturally occurring bases, nucleotide base analogs or other chemical nucleotide modifiers. Typically the length of the sequence is between 2-25 bases, but can range from 2-40 bases or 2-50 bases in length. In example, a polymorphism with two allele-specific primers can have one primer with a 6-base long 5' sequence, and a second primer with a 14-base long 5' sequence. Further, the sequence of the first 5' non-complementary sequence can be 5'-GCGGGC-3' (SEQ ID NO-01), and the sequence of the second 5' non-complementary sequence can be 5'-GCGGGCAGGGCGGC-3' (SEQ ID NO-02). Alternatively, the first 5' non-complementary sequence can be a 3-base long 5' sequence which can be: 5'-GCG-3' (SEQ ID NO-03).

Each 5' non-complementary sequence is unique from other 5' non-complementary sequences in that each has a different melting profile, which is determined both by the strength of the bond (G-C bonds being stronger than A-T bonds), the length of the sequence, the secondary structure of the sequence and possibly the affects occurring from the use of nucleotide base analogs and chemically-modified nucleotide bases. During amplification, the primers are incorporated into the amplification products, or amplicons. Therefore the completed amplicons contain the unique 5' sequences. Amplicons generated from different allele-specific primers will then each contain different unique 5' sequences. A melting curve analysis will determine which 5' unique sequence is contained in the amplicon, thereby determining which nucleotide polymorphism was present in the original sample. In addition to the above described methods of determining a base at a particular nucleotide polymorphism site by measuring the melting temperature of the amplicon, other methods of detection could utilize the inherent features that the amplification products or amplicons created by the above described methods could be separated by size, molecular weight, as well as by melting temperature. In certain embodiments, for example, methods are provided to determine the base at the particular nucleotide polymorphism site by detecting if the amplification reaction products or amplicons are created from the first forward primer or the second forward primer. Methods to distinguish DNA fragments by size are well known in the art.

In the design of the forward primers, to increase the success rate of the amplification reaction performing as expected on the first experimental design trial, it can be helpful to attach the different lengths of 5' non-complementary sequences to specific nucleotide polymorphism forward primers. For example, at a given polymorphism site which comprises either a G or a T nucleotide at the terminal 3' end of the forward primer, a longer 5' non-complementary sequence (for example, a 14bp sequence) can be attached on the forward primer that designed for the stronger-bond nucleotide (G) at the 3' end, and a shorter non-complementary sequence (for example, a 6bp sequence) can be attached to the other primer designed for the weaker-bond nucleotide (T) at the 3' end. Typically the forward primers are present in the amplification reaction at a concentration of approximately 0.2µM. However, if one forward primer results in more efficient amplification than other forward primer, giving rise to uneven results, then one can shift this imbalance by reduction of the primer concentration of the primer that amplifies more efficiently, or by increase of the primer concentration of the primer that amplifies less efficiently. Other standard amplification assay parameters may need optimization, such as adjustments of annealing temperature in PCR, enzyme concentration, metal ion concentration, etc.

The amplification reaction also contains a primer in the reverse direction from the allele-specific primer. This reverse primer is complementary to the sequence downstream of the nucleotide polymorphism site. The reverse primer is typically no more than 20 bp downstream from the nucleotide polymorphism site, resulting in good amplification efficiency and relatively short amplicons in the range of 45 to 70 bp in length. The reverse primer could also be up to 100 bp downstream from the nucleotide polymorphism site, resulting in amplicons greater than 120 bp in length. This reverse primer combined with all forward primers designed at the particular nucleotide polymorphism site can amplify the nucleic acid sample. The resulting amplicons will all have the same 3' end sequence.

In certain embodiments of the methods, the 3' section of each of the forward polymorphism-specific primers, without the 5' non-complementary additional sequence, is designed to have similar Tm characteristics to the other forward primers in the reaction. For example, all 3' sections of forward primers can be designed to have a Tm of approximately 58°C. Then, as described, a unique non-complementary sequence is added to the 5' end of each of the polymorphism-specific primers. The reverse primers may be designed to have a Tm slightly higher than that of the polymorphism-specific portion of the forward primers. For example, if the 3' sections of the forward primers have a Tm of approximately 58°C, then the reverse primer can be designed to have a Tm of approximately 61°C.

The presented methods include the determination of a base at a particular nucleotide polymorphism site in a nucleic acid sample. This nucleic acid sample is typically genomic DNA, but can also be RNA. Typically the DNA or RNA is purified from cell culture extracts, or whole blood, white cells, plasma, or serum, but could also come from other sources such as buccal cells, hairs, or saliva. It is possible that the nucleic acid sample is not purified from its source, and is used in the methods in its original sample state or a semi-purified state.

The amplification reaction also contains a polymerase, which includes a DNA polymerase and may include other polymerases having, for example, reverse transcriptase activity. Specificity of an amplification reaction can be affected by choice of polymerase and related activities. For example, a truncated form ofTaq polymerase, Stoffel DNA polymerase (Lawyer, F.C., S. Stoffel, R.K. Saiki, S.Y. Chang, P.A. Landre, R.D. Abramson, and D.H. Gelfand. 1993. High-level expression, purification, and enzymatic characterization of full-length Thermus aquaticus DNA polymerase and a truncated form deficient in 5' to 3' exonuclease activity. PCR Methods Appl 2: 275-287), has been shown to enhance discrimination of 3' primer-template mismatches (Tada, M., M. Omata, S. Kawai, H. Saisho, M. Ohto, R.K. Saiki, and J.J. Sninsky. 1993. Detection of ras gene mutations in pancreatic juice and peripheral blood of patients with pancreatic adenocarcinoma. Cancer Res 53: 2472-2474).

To further increase the specificity of the amplification reaction, a "hot start" technique may be used. These techniques offer the advantage of decreased non-specific amplification by the reduction of the amount of non-specific reaction in the initial cycle of amplification. To provide a "hot start" to the amplification reaction, for example, polymerase can be added at an elevated temperature; however this can be inconvenient when a large number of samples are amplified. Another common approach to providing a hot start is to utilize a component that prevents non-specific activity in the initial cycle. One example is the use of a chemically modified version of a DNA polymerase, used to provide a simplified hot start to the reaction, as described in US patents US 5,677,152 and US 5,773,258. Polymerase modified in this manner is termed a "Gold" polymerase. The "Gold" polymerase is inactive in the amplification reaction mixture until the mixture is heated for a period of time at an elevated temperature, for example, 95°C for 3-15 minutes. This "Gold" modifier can be utilized with any polymerase, including the Stoffel fragment DNA polymerase, creating a Stoffel "Gold" fragment DNA polymerase. The use of this type of modified polymerase, or the use of another hot start method, can largely decrease the non-specific amplification that may occur in the initial heating steps of the reaction. The hot start activity may also result from the use of other moieties, including for example the use of an aptamer, or an anti-Taq antibody.

The methods describe the use of a double-stranded DNA binding or intercalating dye, such as ethidium bromide or SYBR-green (Molecular Probes). These dyes may be present during the amplification reaction, or they can be added after the amplification reaction is completed. The dyes are used to determine the melting temperature of the amplification products, or amplicons.

The methods presented may be utilized in any primer-mediated amplification technique. Polymerase Chain Reaction, or PCR, is a primer-mediated amplification technique well known in the art. Other primer-mediated amplification techniques include but are not limited to the ligase chain reaction (LCR), linked linear amplification (LLA), and strand displacement amplification (SDA). The methods presented may be performed in a single closed tube, allowing for one-tube detection of multiple polymorphism sites, eliminating complicated post-amplification manipulation, and significantly reducing the risk of amplicon contamination. The volume of the amplification reaction is related to the amplification methods and instruments utilized, and the volume can be 100µl or less, or 50µl or less, or 10µl or less, or 5µl or less.

After amplification, the specific polymorphism is determined, for example, by inspection of a melting curve analysis, which shows which of the primers have amplified. This melting curve analysis may be done utilizing, for example, a real time PCR instrument such as the ABI 5700/7000 (96 well format) or ABI 7900 (384 well format) instrument with onboard software (SDS 2.1). A double-stranded DNA binding dye provides the indicator for determination of the melting temperature of the amplicon. When the amplicon is double-stranded, at lower temperatures, the dye will fluoresce. As the temperature is increased, the amplicon will begin to melt at a rate based on a complex of factors including sequence length, base composition and presence of other nucleotide analogs or modifiers. As the double-stranded amplicon melts to a single strand, the fluorescence of the dye will decrease. The fluorescence intensity of the amplification products is measured from, for example, 60°C to 92°C. The melting analysis values are determined by calculating the negative derivative of the change in fluorescence. Each unique amplicon sequence will result in different melting analysis values.

A desired characteristic found in the use of melting curves is the ability to examine PCR products in an end-point analysis. Since it is not necessary to monitor the fluorescence signals in real-time, any thermal cycler may be used for the amplification steps. In some cases, off-line amplification in a traditional, less expensive thermalcycler enables the better use of the most expensive instrument with fluorescent reading capabilities (for example, the real-time thermal cycler). These methods allow for nucleic acid polymorphism determination in a 384-well format with conventional PCR amplification in a standard 384 block thermalcycler. Multiple 384 plates of samples may be amplified in standard dual-block thermal cyclers (ABI 9700s) and then transferred to the real-time thermal cycler (ABI 7900) to perform the end-point melting curve analysis.

The melting curve analysis may be performed on a real-time instrument, for example the ABI 7900, using the onboard software (SDS 2.1) to calculate the negative derivative of the change in fluorescence. The melting analysis values can be exported from the onboard software as a text file, and imported to a customized file which can be designed to perform a series of normalization and quality control calculations and allow a semi-automated scoring of the polymorphism genotypes. In the first step of the analysis procedure, the fluorescence data for each sample can be normalized by the differences in well-to-well temperature for each fluorescence measurement given. Next the overall data area of interest corresponding to the outer temperature boundaries of the two peaks of the melting curves can be determined, in order to exclude artificial signal arising either from instrument noise or spurious, non-specific amplification (e.g. primer-dimer). Then a center temperature point can be determined that unambiguously separates the two melting curve peaks. A second round of normalization can be performed using this center temperature such that for each sample the maximum peak height can be determined to fall left or right of the peak area, then all samples can be normalized by falling into either area such that the temperature of their peak maxima are normalized to the average temperature for that area (left or right). A quality control algorithm can be implemented which can average the first two data points of the raw fluorescence values (at 60°C) for any sample designated as a no-template control, and can exclude any sample with a lower initial raw fluorescence value from genotype calling. Samples with a lower initial raw fluorescence reading than the highest no-template control at 60°C can be assumed to contain insufficient amounts of PCR amplicon to warrant genotype calling. This calculation can prevent samples that failed amplification from being assigned a genotype based on the melting curve of non-specific amplification products (e.g. primer-dimer). Next the data points of the melting curves can be integrated within each of two peak areas. The analysis software can define default peak areas for integration as +/- 1°C of the average peak maxima for the left and the right peak respectively. It can be necessary to fine tune these peak areas to maximize discrimination and improve clustering. The resulting values for each sample can be graphed in a scatter plot with coordinates corresponding to the integrated peak areas for the first and the second alleles; the arctangent value (y/x) of each point to the origin (0,0) can be calculated where x and y are the two peak integration values. A k-means clustering algorithm may be used to automatically assign the genotypes (Anderberg, M.R. 1973. Cluster analysis for application. Academic Press, New York; and Sharma, S. 1996. Applied Multivariate Techniques. John Willey & Sons, New York which automatically classifies samples into four genotype categories based on nearest-centroid sorting. The algorithm employs the arctangent values of each sample as the metrics used during the clustering process. Samples that are either predefined as non-template controls or may have been filtered by the previously described quality control are not used during clustering. These samples can be assigned a "NA" label. Data are classified into a predetermined number of clusters (e.g. 4 clusters with three genotypes clusters and one NA cluster, or three genotype clusters if the variant homozygote is not present in a particular run), and samples are assigned to the cluster with the smallest distance between the sample data and the center of the cluster (centroid). The polymorphism genotypes are assigned automatically to samples in each cluster.

To further increase the throughput of the genotype calling process, a standalone application has been developed written in Java using the same k-means algorithm as previously described. The application can simultaneously analyze melting curve results from multiple 384-well plate for a given polymorphism site and automatically assign genotypes. The application can flag genotypes that appear to be outliers by noting samples that are shifted more than a 1°C (default value, can be changed as determined empirically) during the temperature normalization. Genotypes can still be assigned to these samples, and the default allowable temperature shift can be increased for assays that have greater variability in peak maxima temperatures. The software can also note samples with a low conditional probability of belonging to a particular cluster. The noted samples can be manually changed or excluded after inspection of melting curves. This use of the 384-well format has resulted in significant increase in the throughput over conventional 96-well thermalcyclers. With a relatively simple laboratory setup, including for example one BioMek 2000, 2 ABI 9700 thermal cyclers, and one ABI 7900HT real-time thermal cycler, one technician can routinely process 8-10 plates for roughly 3,500 polymorphisms per day. This throughput could be improved with a faster liquid handling robot with a 96-probe tool (e.g. the BioMek Fx), additional ABI 9700's, and the use of the automatic loading device plate stacker available on the ABI7900HT. This decreases the necessary hands-on time required for plate processing, to enable a throughput of 6,000-10,000 polymorphisms per day.

### EXAMPLES

### Example 1 - 14 bp and 6 bp 5' regions

PCR reactions are performed on genomic DNA in 100µl reaction volumes. Reaction and cycling conditions are optimized to conditions as described below to minimize non-homologous allele amplification and primer-dimmer formation. A modified ( termed "gold") version of Taq Stoffel fragment DNA polymerase, prepared in-house (as per US 5,677,152 and US 5,773,258), is used to provide a hot start to the reaction. For each nucleotide polymorphism site having 1 polymorphic base site with 2 possible alleles, two forward primers are designed with the terminal 3' base of each primer matching only one of the polymorphic bases. The 5' end of each forward primer comprises GC-rich sequences; one primer having a 14-base-long 5' end non-complementary to the target DNA and the second primer having a 6-base-long 5' end non-complementary to the target DNA (see underlined sequence, TABLE 1). Common reverse primers are designed downstream of each polymorphic site. Each polymorphism site is analyzed in a separate reaction. The polymorphisms (SNPs) are noted by name or by GenBank accession numbers as of June 13, 2003, which correlate to the following reference cluster (rs) numbers from the NCBI human SNP (dbSNP) database as of April 21, 2005: ApoB71 = rs1367117, AC006408.1_51078 = rs734351, AC006408.1_45311 = rs2585, AC006408.1_65008 = rs1004446.

**TABLE 1**

| Polymorphism | Primer Name | Description | Sequence 5'-3' |
|---|---|---|---|
| AC006408.1_ 51078 | MA1834 | Allele 1 | GCGGGCAGGGCGGCTGCACCTTTTGGACATT C (SEQ ID NO-04) |
| | MA1828 | Allele 2 | GCGGGCTGCACCTTTTGGACATTT (SEQ ID NO-05) |
| | SG1819 | Common | GAACGCACGTCCCTTGTC (SEQ ID NO-06) |
| AC006408.1_ 45311 | MA1836 | Allele 1 | GCGGGCAGGGCGGCCCGTGTTTGACTCAACT CAG (SEQ ID NO-07) |
| | MA1829 | Allele 2 | GCGGGCCCGTGTTTGACTCAACTCAA (SEQ ID NO-08) |
| | SG1792 | Common | TTTGCCGGAAATATTAGCGT (SEQ ID NO-09) |
| AC006408.1_ 65008 | MA1846 | Allele 1 | GCGGGCAGGGCGGCTTGTGCAGCCCTAAAGG (SEQ ID NO-10) |
| | MA1830 | Allele 2 | GCGGGCTTGTGCAGCCCTAAAGA (SEQ ID NO-11) |
| | SG1849 | Common | CAGGAAAGGCCATTGTGGAGA (SEQ ID NO-12) |
| ApoB71 | MA1852 | Allele 1 | GCGGGCAGGGCGGCGAAGACCAGCCAGTGC AC (SEQ ID NO-13) |
| | MA1850 | Allele 2 | GCGGGCGAAGACCAGCCAGTGCAT (SEQ ID NO-14) |
| | SC1272B | Common | CAAGGCTTTGCCCTCAGGGTT (SEQ ID NO-15) |

PCR reactions contain the following, in final concentration: 0.2 µM each primer; 12 units of Stoffel "Gold" polymerase; 2 units of UNG (uracil N-glycosylase); 10mM Tris-HCL, 40mM KCl at pH 8.0; 2mM MgCl₂; 50 µM each dATP, dCTP, and dGTP; 25 µM dTTP; 75 µM dUTP; 0.2 X SYBR Green I (Molecular Probes); 5% DMSO; 2.2% glycerol; 2.0 µM ROX dye (Molecular Probes). 20 ng of genomic DNA (purified from cell lines using standard methods) is added to each reaction.

PCR is performed on an ABI 5700 instrument (Applied Biosystems). Two initial heating steps are performed: 2 minutes at 50°C to activate the UNG, followed by 12 minutes at 95°C to activate the "Gold" polymerase. 45 cycles are performed of three-step amplification of 20 seconds at 95°C, 40 seconds at 58°C and 20 seconds at 72°C.

After amplification, the melting curve analysis is performed on the ABI 5700 instrument. The fluorescence intensity of the PCR product is measured from 60°C to 92°C. The melting analysis values are exported from the onboard software as a text file, and imported to a custom MS Excel spreadsheet. Melting curves are generated by plotting the negative derivative of the change in fluorescence against the temperature for each reaction. For each individual polymorphism site, the melting curve peak in the lower temperature range corresponds to amplification with the forward primer which has the shorter, 6-base 5' non-complementary sequence attached. The melting curve peak in the higher temperature range corresponds to amplification with the other forward primer which has the longer, 14-base 5' non-complementary sequence attached. When the melting curve peaks fall into both temperature ranges, then amplification with both forward primers is indicated.

### Example 2 - 10 bp and 3 bp 5' regions

PCR is performed as per the reaction conditions and analysis methods as described above in Example 1, with the following modifications. PCR reactions are performed with 10ng of genomic DNA in 50µl reaction volumes. The 5' end of each forward primer comprises GC-rich sequences, one primer having a 10-base-long 5' end non-complementary to the target DNA and the second primer having a 3-base-long 5' end non-complementary to the target DNA (see underlined sequence, TABLE 2).

**TABLE2**

| Polymorphism | Primer Name | Description | Sequence 5'-3' |
|---|---|---|---|
| AC006408.1_51078 | JWG1202 | Allele 1 | GCGGGCAGGGTGCACCTTTTGGAC ATTC (SEQ ID NO-16) |
| | JWG 1203 | Allele 2 | GCGTGCACCTTTTGGACATTT (SEQ ID NO-17) |
| | JWG1204 | Common | GAACGCACGTCCCTTGTC (SEQ ID NO-18) |
| AC006408.1_45311 | JWG1205 | Allele 1 | GCGGGCAGGGCCGTGTTTGACTCA ACTCAG (SEQ ID NO-19) |
| | JWG1206 | Allele 2 | GCGCCGTGTTTGACTCAACTCAA (SEQ ID NO-20) |
| | JWG1207 | Common | TTTGCCGGAAATATTAGCGT (SEQ ID NO-21) |
| AC006408.1_65008 | JWG1208 | Allele 1 | GCGGGCAGGGTTGTGCAGCCCTAA AGG (SEQ ID NO-22) |
| | JWG1209 | Allele 2 | GCGTTGTGCAGCCCTAAAGA (SEQ ID NO-23) |
| | JWG1210 | Common | CAGGAAAGGCCATTGTGGAGA (SEQ ID NO-24) |
| ApoB71 | JWG1211 | Allele 1 | GCGGGCAGGGGAAGACCAGCCAGT GCAC (SEQ ID NO-25) |
| | JWG1212 | Allele 2 | GCGGAAGACCAGCCAGTGCAT (SEQ ID NO-26) |
| | JWG1213 | Common | CAAGGCTTTGCCCTCAGGGTT (SEQ ID NO-27) |

For each individual polymorphism site, the melting curve peak in the lower temperature range corresponds to amplification with the forward primer which has the shorter, 3-base 5' non-complementary sequence attached. The melting curve peak in the higher temperature range corresponds to amplification with the other forward primer which has the longer, 10-base 5' non-complementary sequence attached. When the melting curve peaks fall into both temperature ranges, then amplification with both forward primers is indicated.

### Example 3 - 26 bp and 18 bp 5' regions

PCR is performed as per the reaction conditions and analysis methods as described above in Example 1, with the following modifications. PCR reactions are performed with 10ng of genomic DNA in 50µl reaction volumes. The 5' end of each forward primer comprises GC-rich sequences; one primer having a 26-base-long 5' end non-complementary to the target DNA and the second primer having an 18-base-long 5' end non-complementary to the target DNA (see underlined sequence, TABLE 3).

**TABLE3**

| Polymorphism | Primer Name | Description | Sequence 5'-3' |
|---|---|---|---|
| AC006408.1_51078 | JWG 1214 | Allele 1 | GCGGGCAGGGCGGCGGGGGCGGGGC CTGCACCTTTTGGACATTC (SEQ ID NO-28) |
| | JWG1215 | Allele 2 | GCGGGCAGGGCGGCGGGGTGCACCTT TTGGACATTT (SEQ ID NO-29) |
| | JWG1216 | Common | GAACGCACGTCCCTTGTC (SEQ ID NO-30) |
| AC006408.1_45311 | JWG1217 | Allele 1 | GCGGGCAGGGCGGCGGGGGCGGGGC CCCGTGTTTGACTCAACTCAG (SEQ ID NO-31) |
| | JWG1218 | Allele 2 | GCGGGCAGGGCGGCGGGGCCGTGTTT GACTCAACTCAA (SEQ ID NO-32) |
| | JWG1219 | Common | TTTGCCGGAAATATTAGCGT (SEQ ID NO-33) |
| AC006408.1_65008 | JWG1220 | Allele 1 | GCGGGCAGGGCGGCGGGGGCGGGGC CTTGTGCAGCCCTAAAGG (SEQ ID NO-34) |
| | JWG 1221 | Allele 2 | GCGGGCAGGGCGGCGGGGTTGTGCA GCCCTAAAGA (SEQ ID NO-35) |
| | JWG1222 | Common | CAGGAAAGGCCATTGTGGAGA (SEQ ID NO-36) |
| ApoB71 | JWG 1223 | Allele 1 | GCGGGCAGGGCGGCGGGGGCGGGGC CGAAGACCAGCCAGTGCAC (SEQ ID NO-37) |
| | JWG1224 | Allele 2 | GCGGGCAGGGCGGCGGGGGAAGACC AGCCAGTGCAT (SEQ ID NO-38) |
| | JWG1225 | Common | CAAGGCTTTGCCCTCAGGGTT (SEQ ID NO-39) |

For each individual polymorphism site, the melting curve peak in the lower temperature range corresponds to amplification with the forward primer which has the shorter, 18-base 5' non-complementary sequence attached. The melting curve peak in the higher temperature range corresponds to amplification with the other forward primer which has the longer, 26-base 5' non-complementary sequence attached. When the melting curve peaks fall into both temperature ranges, then amplification with both forward primers is indicated.

### Example 4 - 14bp and 6bp 5'regions with penultimate 3' base

PCR is performed as per the reaction conditions and analysis methods as described above in Example 1, with the following modifications. PCR reactions are performed with 10ng of genomic DNA in 50µl reaction volumes. PCR is performed on an ABI 5700 instrument (Applied Biosystems). Two initial heating steps are performed: 2 minutes at 50°C to activate the UNG, followed by 12 minutes at 95°C to activate the Gold polymerase. 38 cycles of three-step amplification of 20 seconds at 95°C, 40 seconds at 65°C and 20 seconds at 72°C are performed. Two forward primers are designed with the penultimate 3' base of each primer matching only one of the polymorphic bases. The 5' end of each forward primer comprises GC-rich sequences; one primer having a 14-base-long 5' end non-complementary to the target DNA and the second primer having a 6-base-long 5' end non-complementary to the target DNA (see underlined sequence, TABLE 4).

**TABLE4**

| Polymorphism | Primer Name | Description | Sequence 5'-3' |
|---|---|---|---|
| AC006408.1_51078 | JWG1238 | Allele 1 | GCGGGCAGGGCGGCTGCACCTTTTGG ACATTCT (SEQ ID NO-40) |
| | JWG1239 | Allele 2 | GCGGGCTGCACCTTTTGGACATTTT (SEQ ID NO-41) |
| | JWG1240 | Common | GAACGCACGTCCCTTGT (SEQ ID NO-42) |
| AC006408.1_45311 | JWG1241 | Allele 1 | GCGGGCAGGGCGGCCCGTGTTTGACT CAACTCAGC (SEQ ID NO-43) |
| | JWG1242 | Allele 2 | GCGGGCCCGTGTTTGACTCAACTCAA C (SEQ ID NO-44) |
| | JWG1243 | Common | TTTGCCGGAAATATTAGCGT (SEQ ID NO-45) |
| AC006408.1_65008 | JWG1244 | Allele 1 | GCGGGCAGGGCGGCTTGTGCAGCCCT AAAGGA (SEQ ID NO-46) |
| | JWG1245 | Allele 2 | GCGGGCTTGTGCAGCCCTAAAGAA (SEQ ID NO-47) |
| | JWG1246 | Common | CAGGAAAGGCCATTGTGGAGA (SEQ ID NO-48) |
| ApoB71 | JWG1247 | Allele 1 | GCGGGCAGGGCGGCGAAGACCAGCC AGTGCACC (SEQ ID NO-49) |
| | JWG1248 | Allele 2 | GCGGGCGAAGACCAGCCAGTGCATC (SEQ ID NO-50) |
| | JWG1249 | Common | CAAGGCTTTGCCCTCAGGGTT (SEQ ID NO-51) |

For each individual polymorphism site, the melting curve peak in the lower temperature range corresponds to amplification with the forward primer which has the shorter, 6-base 5' non-complementary sequence attached. The melting curve peak in the higher temperature range corresponds to amplification with the other forward primer which has the longer, 14-base 5' non-complementary sequence attached. When the melting curve peaks fall into both temperature ranges, then amplification with both forward primers is indicated.

### Example 5 - Multiplex detection of 2 polymorphic sites

PCR is performed as per the reaction conditions and analysis methods as described above in Example 1, with the following modifications. PCR reactions are performed with 10ng of genomic DNA in 50µl reaction volumes. PCR is performed on an ABI 5700 instrument (Applied Biosystems). Two initial heating steps are performed: 2 minutes at 50°C to activate the UNG, followed by 12 minutes at 95°C to activate the Gold polymerase. 40 cycles of three-step amplification of 20 seconds at 95°C, 40 seconds at 58°C and 20 seconds at 72°C are performed. Primers sets for 2 polymorphic sites are combined into one reaction tube. For each polymorphic site two forward primers are designed, for a total of four forward primers. The 5' end of each forward primer comprises 5' non-complementary GC-rich sequences, the first primer having a 10-base-long 5' end, the second primer having a 3-base-long 5' end, the third primer having a 26-base-long 5' end, and the fourth primer having an 18-base-long 5' end (see **underlined** sequence, **TABLE 5**).

**TABLE 5**

| Polymorphism | Primer Name | Description | Sequence 5'-3' |
|---|---|---|---|
| AC006408.1_51078 | JWG 1202 | Allele 1 | GCGGGCAGGGTGCACCTTTTGGACAT TC (SEQ ID NO-16) |
| | JWG1203 | Allele 2 | GCGTGCACCTTTTGGACATTT (SEQ ID NO-17) |
| | JWG1204 | Common | GAACGCACGTCCCTTGTC (SEQ ID NO-18) |
| ApoB71 | JWG1223 | Allele 1 | GCGGGCAGGGCGGCGGGGGCGGGGC CGAAGACCAGCCAGTGCAC (SEQ ID NO-37) |
| | JWG1224 | Allele 2 | GCGGGCAGGGCGGCGGGGGAAGACC AGCCAGTGCAT (SEQ ID NO-38) |
| | JWG1225 | Common | CAAGGCTTTGCCCTCAGGGTT (SEQ ID NO-39) |

For the multiplex reaction of the two polymorphism sites, the amplification can result in 4 unique peaks. The melting curve peak in the lowest temperature range corresponds to amplification with the first forward primer which has the shortest, 3-base 5' non-complementary sequence attached. The melting curve peak in the second to lowest temperature range corresponds to amplification with the second forward primer which has the 10-base 5' non-complementary sequence attached. The melting curve peak in the second to highest temperature range corresponds to amplification with the third forward primer which has the 18-base 5' non-complementary sequence attached. The melting curve peak in the highest temperature range corresponds to amplification with the fourth forward primer which has the longest, 26-base 5' non-complementary sequence attached. When the melting curve peaks fall into multiple temperature ranges, then amplification with two, three or all four forward primers is indicated.

### Example 6 - 14 bp and 3 bp 5' regions

PCR is performed as per the reaction conditions and analysis methods as described above in Example 1, with the following modifications. PCR reactions are performed with 4ng of genomic DNA in 15µl reaction volumes. 1.8 units of Stoffel Gold is used; no UNG is added to the reaction mix. ROX dye is added at 0.25 µM. The 5' end of each forward primer comprises GC-rich sequences; one primer having a 14-base-long 5' end non-complementary to the target DNA and the second primer having a 3-base-long 5' end non-complementary to the target DNA (see underlined sequence, TABLE 6). A common reverse primer is designed downstream of the polymorphic site. PCR is performed on an ABI 7900HT Fast Real-Time PCR System instrument (Applied Biosystems). Two initial heating steps are performed: 2 minutes at 50°C, followed by 12 minutes at 95°C to activate the Gold polymerase. 35 cycles of three-step amplification of 20 seconds at 95°C, 60 seconds at 58°C and 30 seconds at 72°C are performed. The polymorphism ESR1_rs746432 is the reference cluster (rs) number from the NCBI human SNP (dbSNP) database as of April 21, 2005.

**TABLE6**

| Polymorphism | Primer Name | Description | Sequence 5'-3' |
|---|---|---|---|
| ESRI_rs746432 | JWG1355 | Allele 1 | GCGGGCAGGGCGGCGGGTCTGAGGC TGCG (SEQ ID NO-52) |
| | JWG1356 | Allele 2 | GCGGGGTCTGAGGCTGCC (SEQ ID NO-53) |
| | JWG1357 | Common | AGGCCGTTGGAGCCGAAC (SEQ ID NO-54) |

For each polymorphism site, the melting curve peak in the lower temperature range corresponds to amplification with the forward primer which has the shorter, 3-base 5' non-complementary sequence attached. The melting curve peak in the higher temperature range corresponds to amplification with the other forward primer which has the longer, 14-base 5' non-complementary sequence attached. When the melting curve peaks fall into both temperature ranges, then amplification with both forward primers is indicated.

It is understood that the examples and embodiments described herein are for illustrative purposes only and are not intended to limit the scope of the claimed invention.

## Claims

1. A method to determine a base at a particular nucleotide polymorphism site in a nucleic acid sample comprising:
a) amplifying the nucleic acid sample in an amplification reaction comprising:
a first forward primer comprising a 3' terminal base complementary to a first base at the particular nucleotide polymorphism site and a first 5' non-complementary sequence,
a second forward primer comprising a 3' terminal base complementary to a second base at the particular nucleotide polymorphism site and a second 5' non-complementary sequence that is different in melting temperature than the first 5' non-complementary sequence,
a reverse primer complementary to sequence downstream of the particular nucleotide polymorphism site, and
a DNA polymerase;
b) creating an amplification reaction product;
c) measuring the melting temperature of the amplification reaction product; and
d) determining the base at the particular nucleotide polymorphism site by the melting temperature of the amplification reaction product.

2. The method of claim 1, wherein the amplification reaction further comprises: a third forward primer comprising a 3' terminal base complementary to a third base at the particular nucleotide polymorphism site, and a third 5' non-complementary sequence that is different in melting temperature than the first and second 5' non-complementary sequences.

3. The method of claim 2, wherein the amplification reaction further comprises: a fourth forward primer comprising a 3' terminal base complementary to a fourth base at the particular nucleotide polymorphism site, and a fourth 5' non-complementary sequence that is different in melting temperature than the first and second and third 5' non-complementary sequences.

4. A method to determine a base at a particular nucleotide polymorphism site in a nucleic acid sample comprising:
a) amplifying the nucleic acid sample in an amplification reaction comprising:
a first forward primer comprising a penultimate 3' base complementary to a first base at the particular nucleotide polymorphism site and a first 5' non-complementary sequence,
a second forward primer comprising a penultimate 3' base complementary to a second base at the particular nucleotide polymorphism site and a second 5' non-complementary sequence that is different in melting temperature than the first 5' non-complementary sequence,
a reverse primer complementary to sequence downstream of the particular nucleotide polymorphism site, and
a DNA polymerase;
b) creating an amplification reaction product;
c) measuring the melting temperature of the amplification reaction product; and
d) determining the base at the particular nucleotide polymorphism site by the melting temperature of the amplification reaction product.

5. The method of claim 4, wherein the amplification reaction further comprises: a third forward primer comprising a penultimate 3' base complementary to a third base at the particular nucleotide polymorphism site, and a third 5' non-complementary sequence that is different in melting temperature than the first and second 5' non-complementary sequences.

6. The method of claim 5, wherein the amplification reaction further comprises: a fourth forward primer comprising a penultimate 3' base complementary to a fourth base at the particular nucleotide polymorphism site, and a fourth 5' non-complementary sequence that is different in melting temperature than the first and second and third 5' non-complementary sequences.

7. The method as in claim 1, wherein the amplifying the nucleic acid sample, the creating the amplification reaction product, and the measuring the melting temperature are carried out in a single tube.

8. The method as in claim 1, wherein the 5' non-complementary sequences comprise at least one of:
a) nucleotide base analogs, and
b) chemically-modified nucleotide bases.

9. The method as in claim 1, wherein the 5' non-complementary sequences are from 2 base pairs to 25 base pairs in length.

10. The method as in claim 1, wherein the first 5' non-complementary sequence is 3 base pairs in length and the second 5' non-complementary sequence is 14 base pairs in length.

11. The method as in claim 1, wherein the sequence of the first 5' non-complementary sequence is
5'-GCG -3' as in SEQ ID NO-03
and the sequence of the second 5' non-complementary sequence is
5'-GCGGGCAGGGCGGC-3' as in SEQ ID NO-02.

12. The method as in claim 1, wherein the DNA polymerase lacks 5'-3' exonuclease activity.

13. The method as in claim 1, wherein the DNA polymerase is modified to have hot start activity.

14. The method as in claim 1, wherein the amplification reaction contains at least one of:
a) a fluorescent double-stranded nucleic acid binding dye, and
b) an intercalating dye.

15. A method to determine a base at a particular nucleotide polymorphism site in a nucleic acid sample comprising:
a) amplifying the nucleic acid sample in an amplification reaction comprising:
a first forward primer comprising a 3' terminal base complementary to a first base at the particular nucleotide polymorphism site and a first 5' non-complementary sequence,
a second forward primer comprising a 3' terminal base complementary to a second base at the particular nucleotide polymorphism site and a second 5' non-complementary sequence that is different in melting temperature than the first 5' non-complementary sequence,
a reverse primer complementary to sequence downstream of the particular nucleotide polymorphism site, and
a DNA polymerase;
b) creating an amplification reaction product; and
c) determining the base at the particular nucleotide polymorphism site by detecting if the amplification reaction product is created from the first forward primer or the second forward primer.

## Patentansprüche

1. Verfahren zur Bestimmung einer Base an einer bestimmten Nukleotidpolymorphismusstelle in einer Nukleinsäureprobe, bei dem man
a) die Nukleinsäureprobe in einer Amplifikationsreaktion, die
einen ersten Vorwärtsprimer mit einer zu einer ersten Base an der bestimmten Nukleotidpolymorphismusstelle komplementären 3'-terminalen Base und einer ersten 5'-nichtkomplementären Sequenz,
einen zweiten Vorwärtsprimer mit einer zu einer zweiten Base an der bestimmten Nukleotidpolymorphismusstelle komplementären 3'-terminalen Base und einer zweiten 5'-nichtkomplementären Sequenz, die sich von der ersten 5'-nichtkomplementären Sequenz hinsichtlich der Schmelztemperatur unterscheidet,
einen zu stromabwärts von der bestimmten Nukleotidpolymorphismusstelle liegender Sequenz komplementären Rückwärtsprimer und
eine DNA-Polymerase
umfasst, amplifiziert,
b) ein Amplifikationsreaktionsprodukt erzeugt,
c) die Schmelztemperatur des Amplifikationsreaktionsprodukts misst und
d) die Base an der bestimmten Nukleotidpolymorphismusstelle über die Schmelztemperatur des Amplifikationsreaktionsprodukts bestimmt.

2. Verfahren nach Anspruch 1, wobei die Amplifikationsreaktion ferner
einen dritten Vorwärtsprimer mit einer zu einer dritten Base an der bestimmten Nukleotidpolymorphismusstelle komplementären 3'-terminalen Base und einer dritten 5'-nichtkomplementären Sequenz, die sich von der ersten und der zweiten 5'-nichtkomplementären Sequenz hinsichtlich der Schmelztemperatur unterscheidet, umfasst.

3. Verfahren nach Anspruch 2, wobei die Amplifikationsreaktion ferner
einen vierten Vorwärtsprimer mit einer zu einer vierten Base an der bestimmten Nukleotidpolymorphismusstelle komplementären 3'-terminalen Base und einer vierten 5'-nichtkomplementären Sequenz, die sich von der ersten und der zweiten und der dritten 5'-nichtkomplementären Sequenz hinsichtlich der Schmelztemperatur unterscheidet, umfasst.

4. Verfahren zur Bestimmung einer Base an einer bestimmten Nukleotidpolymorphismusstelle in einer Nukleinsäureprobe, bei dem man
a) die Nukleinsäureprobe in einer Amplifikationsreaktion, die
einen ersten Vorwärtsprimer mit einer zu einer ersten Base an der bestimmten Nukleotidpolymorphismusstelle komplementären vorletzten 3'-Base und einer ersten 5'-nichtkomplementären Sequenz,
einen zweiten Vorwärtsprimer mit einer zu einer zweiten Base an der bestimmten Nukleotidpolymorphismusstelle komplementären vorletzten 3'-Base und einer zweiten 5'-nichtkomplementären Sequenz, die sich von der ersten 5'-nichtkomplementären Sequenz hinsichtlich der Schmelztemperatur unterscheidet,
einen zu stromabwärts von der bestimmten Nukleotidpolymorphismusstelle liegender Sequenz komplementären Rückwärtsprimer und
eine DNA-Polymerase
umfasst, amplifiziert,
b) ein Amplifikationsreaktionsprodukt erzeugt,
c) die Schmelztemperatur des Amplifikationsreaktionsprodukts misst und
d) die Base an der bestimmten Nukleotidpolymorphismusstelle über die Schmelztemperatur des Amplifikationsreaktionsprodukts bestimmt.

5. Verfahren nach Anspruch 4, wobei die Amplifikationsreaktion ferner
einen dritten Vorwärtsprimer mit einer zu einer dritten Base an der bestimmten Nukleotidpolymorphismusstelle komplementären vorletzten 3'-Base und einer dritten 5'-nichtkomplementären Sequenz, die sich von der ersten und der zweiten 5'-nichtkomplementären Sequenz hinsichtlich der Schmelztemperatur unterscheidet, umfasst.

6. Verfahren nach Anspruch 5, wobei die Amplifikationsreaktion ferner
einen vierten Vorwärtsprimer mit einer zu einer vierten Base an der bestimmten Nukleotidpolymorphismusstelle komplementären vorletzten 3'-Base und einer vierten 5'-nichtkomplementären Sequenz, die sich von der ersten und der zweiten und der dritten 5'-nichtkomplementären Sequenz hinsichtlich der Schmelztemperatur unterscheidet, umfasst.

7. Verfahren gemäß Anspruch 1, wobei man die Amplifikation der Nukleinsäureprobe, die Erzeugung des Amplifikationsreaktionsprodukts und die Messung der Schmelztemperatur in einem einzigen Röhrchen durchführt.

8. Verfahren gemäß Anspruch 1, wobei die 5'-nichtkomplementären Sequenzen wenigstens entweder
a) Nukleotidbasenanaloge oder
b) chemisch modifizierte Nukleotidbasen
umfassen.

9. Verfahren gemäß Anspruch 1, wobei die 5'-nichtkomplementären Sequenzen eine Länge von 2 Basenpaaren bis 25 Basenpaaren aufweisen.

10. Verfahren gemäß Anspruch 1, wobei die erste 5'-nichtkomplementäre Sequenz eine Länge von 3 Basenpaaren und die zweite 5'-nichtkomplementäre Sequenz eine Länge von 14 Basenpaaren aufweist.

11. Verfahren gemäß Anspruch 1, wobei es sich bei der Sequenz der ersten 5'-nichtkomplementären Sequenz um
5'-GCG-3' gemäß SEQ ID NO-03
und bei der Sequenz der zweiten 5'-nichtkomplementären Sequenz um
5'-GCGGGCAGGGCGGC-3' gemäß SEQ ID NO-02
handelt.

12. Verfahren gemäß Anspruch 1, wobei der DNA-Polymerase 5'-3'-Exonuklease-Aktivität fehlt.

13. Verfahren gemäß Anspruch 1, wobei die DNA-Polymerase modifiziert wird, so dass sie Hot-Start-Aktivität aufweist.

14. Verfahren gemäß Anspruch 1, wobei die Amplifikationsreaktion wenigstens entweder
a) einen doppelsträngige Nukleinsäure bindenden Fluoreszenzfarbstoff oder
b) einen interkalierenden Farbstoff
enthält.

15. Verfahren zur Bestimmung einer Base an einer bestimmten Nukleotidpolymorphismusstelle in einer Nukleinsäureprobe, bei dem man
a) die Nukleinsäureprobe in einer Amplifikationsreaktion, die
einen ersten Vorwärtsprimer mit einer zu einer ersten Base an der bestimmten Nukleotidpolymorphismusstelle komplementären 3'-terminalen Base und einer ersten 5'-nichtkomplementären Sequenz,
einen zweiten Vorwärtsprimer mit einer zu einer zweiten Base an der bestimmten Nukleotidpolymorphismusstelle komplementären 3'-terminalen Base und einer zweiten 5'-nichtkomplementären Sequenz, die sich von der ersten 5'-nichtkomplementären Sequenz hinsichtlich der Schmelztemperatur unterscheidet,
einen zu stromabwärts von der bestimmten Nukleotidpolymorphismusstelle liegender Sequenz komplementären Rückwärtsprimer und
eine DNA-Polymerase
umfasst, amplifiziert,
b) ein Amplifikationsreaktionsprodukt erzeugt und
c) die Base an der bestimmten Nukleotidpolymorphismusstelle über den Nachweis, ob das Amplifikationsreaktionsprodukt von dem ersten Vorwärtsprimer aus oder von dem zweiten Vorwärtsprimer aus erzeugt wird, bestimmt.

## Revendications

1. Procédé de détermination d'une base au niveau d'un site particulier de polymorphisme nucléotidique dans un échantillon d'acide nucléique comprenant :
a) l'amplification de l'échantillon d'acide nucléique dans une réaction d'amplification comprenant :
une première amorce motrice comprenant une base terminale en position 3' complémentaire d'une première base au niveau du site particulier de polymorphisme nucléotidique et une première séquence non complémentaire en position 5',
une seconde amorce motrice comprenant une base terminale en position 3' complémentaire d'une seconde base au niveau du site particulier de polymorphisme nucléotidique et une seconde séquence non complémentaire 5' qui est différente au niveau de la température de fusion par rapport à la première séquence non complémentaire 5', une amorce inverse complémentaire de la séquence en aval du site particulier de polymorphisme nucléotidique, et
une ADN polymérase ;
b) la création d'un produit de réaction d'amplification ;
c) la mesure de la température de fusion du produit de réaction d'amplification ; et
d) la détermination de la base au niveau du site particulier de polymorphisme nucléotidique par la température de fusion du produit de réaction d'amplification.

2. Procédé selon la revendication 1, dans lequel la réaction d'amplification comprend en outre :
une troisième amorce motrice comprenant une base terminale en position 3' complémentaire d'une troisième base au site particulier de polymorphisme nucléotidique, et une troisième séquence non complémentaire 5' qui est différente au niveau de la température de fusion de celle de la première et de la seconde séquences non complémentaires 5'.

3. Procédé selon la revendication 2, dans lequel la réaction d'amplification comprend en outre :
une quatrième amorce motrice comprenant une base terminale en position 3' complémentaire d'une quatrième base au niveau du site particulier de polymorphisme nucléotidique, et une quatrième séquence non complémentaire 5' qui est différente au niveau de la température de fusion de la première et de la seconde et de la troisième séquences non complémentaires 5'.

4. Procédé de détermination d'une base au niveau d'un site particulier de polymorphisme nucléotidique dans un échantillon d'acide nucléique comprenant :
a) l'amplification de l'échantillon d'acide nucléique dans une réaction d'amplification comprenant :
une première amorce motrice comprenant une avant-dernière base 3' complémentaire d'une première base au niveau du site particulier de polymorphisme nucléotidique et une première séquence non complémentaire 5',
une seconde amorce motrice comprenant une avant-dernière base 3' complémentaire d'une seconde base au niveau du site particulier de polymorphisme nucléotidique et une seconde séquence non complémentaire 5' qui est différente au niveau de la température de fusion de la première séquence non complémentaire 5',
une amorce inverse complémentaire de la séquence en aval du site particulier de polymorphisme nucléotidique, et
une ADN polymérase ;
b) la création d'un produit de réaction d'amplification ;
c) la mesure de la température de fusion du produit de réaction d'amplification ; et
d) la détermination de la base au niveau du site particulier de polymorphisme nucléotidique par la température de fusion du produit de réaction d'amplification.

5. Procédé selon la revendication 4, dans lequel la réaction d'amplification comprend en outre :
une troisième amorce motrice comprenant une avant-dernière base 3' complémentaire d'une troisième base au niveau du site particulier de polymorphisme nucléotidique, et une troisième séquence non complémentaire 5' qui est différente au niveau de la température de fusion de la première et de la seconde séquences non complémentaires 5'.

6. Procédé selon la revendication 5, dans lequel la réaction d'amplification comprend en outre :
une quatrième amorce motrice comprenant une avant-dernière base 3' complémentaire d'une quatrième base au niveau du site particulier de polymorphisme nucléotidique, et une quatrième séquence non complémentaire 5' qui est différente au niveau de la température de fusion de la première et de la seconde et de la troisième séquences non complémentaires 5'.

7. Procédé selon la revendication 1, dans lequel l'amplification de l'échantillon d'acide nucléique, la création du produit de réaction d'amplification, et la mesure de la température de fusion sont conduites dans un tube unique.

8. Procédé selon la revendication 1, dans lequel les séquences non complémentaires 5' comprennent au moins l'un parmi :
a) les analogues de base nucléotidique, et
b) des bases nucléotidiques chimiquement modifiées.

9. Procédé selon la revendication 1, dans lequel les séquences non complémentaires 5' sont de deux paires de bases jusqu'à 25 paires de bases de longueur.

10. Procédé selon la revendication 1, dans lequel la première séquence non complémentaire 5' est de 3 paires de bases de longueur et la seconde séquence non complémentaire 5' est de 14 paires de bases de longueur.

11. Procédé selon la revendication 1, dans lequel la séquence de la première séquence non complémentaire 5' est 5'-GCG-3' comme dans SEQ ID NO-3 et la séquence de la seconde séquence non complémentaire 5' est 5'-GCGGGCAGGGCGGC-3' comme dans SEQ ID NO-2.

12. Procédé selon la revendication 1, dans lequel l'ADN polymérase ne possède pas d'activité d'exonucléase 5' vers 3'.

13. Procédé selon la revendication 1, dans lequel l'ADN polymérase est modifiée pour avoir une activité de démarrage à chaud.

14. Procédé selon la revendication 1, dans lequel la réaction d'amplification contient au moins l'un parmi :
a) un colorant fluorescent de liaison à un acide nucléique double brin, et
b) un colorant d'intercalation.

15. Procédé de détermination d'une base au niveau d'un site particulier de polymorphisme nucléotidique dans un échantillon d'acide nucléique comprenant :
a) l'amplification de l'échantillon d'acide nucléique dans une réaction d'amplification comprenant :
une première amorce motrice comprenant une base terminale en position 3' complémentaire d'une première base au niveau du site particulier de polymorphisme nucléotidique et une première séquence non complémentaire 5',
une seconde amorce motrice comprenant une base terminale en position 3' complémentaire d'une seconde base au niveau du site particulier de polymorphisme nucléotidique et une seconde séquence non complémentaire 5' qui est différente au niveau de la température de fusion de la première séquence non complémentaire 5',
une amorce inverse complémentaire de la séquence en aval du site particulier de polymorphisme nucléotidique, et
une ADN polymérase ;
b) la création d'un produit de réaction d'amplification ; et
c) la détermination de la base au niveau du site particulier de polymorphisme nucléotidique en détectant si le produit de réaction d'amplification est créé à partir de la première amorce motrice ou de la seconde amorce motrice.


### SEQUENCE LISTING

<110> F. Hoffmann-La Roche AG
   Grenzacherstrasse 124
   4070 Basel
   Switzerland
<120> METHODS OF GENOTYPING USING DIFFERENCES IN MELTING TEMPERATURE
<130> 22479
<150> 60/647,948
   <151> 2005-01-28
<150> 60/660,187
   <151> 2005-03-09
<160> 54
<170> PatentIn version 3.3
<210> 1
   <211> 6
   <212> DNA
   <213> Artificial
<220>
   <223> 5' Sequence
<400> 1
   gcgggc 6
<210> 2
   <211> 14
   <212> DNA
   <213> Artificial
<220>
   <223> 5' Sequence
<400> 2
   gcgggcaggg cggc 14
<210> 3
   <211> 3
   <212> DNA
   <213> Artificial
<220>
   <223> 5' Sequence
<400> 3
   gcg 3
<210> 4
   <211> 32
   <212> DNA
   <213> Artificial
<220>
   <223> Genotyping Primers
<400> 4
   gcgggcaggg cggctgcacc ttttggacat tc 32
<210> 5
   <211> 24
   <212> DNA
   <213> Artificial
<220>
   <223> Genotyping Primers
<400> 5
   gcgggctgca ccttttggac attt 24
<210> 6
   <211> 18
   <212> DNA
   <213> Artificial
<220>
   <223> Genotyping Primers
<400> 6
   gaacgcacgt cccttgtc 18
<210> 7
   <211> 34
   <212> DNA
   <213> Artificial
<220>
   <223> Genotyping Primers
<400> 7
   gcgggcaggg cggcccgtgt ttgactcaac tcag 34
<210> 8
   <211> 26
   <212> DNA
   <213> Artificial
<220>
   <223> Genotyping Primers
<400> 8
   gcgggcccgt gtttgactca actcaa 26
<210> 9
   <211> 20
   <212> DNA
   <213> Artificial
<220>
   <223> Genotyping Primers
<400> 9
   tttgccggaa atattagcgt 20
<210> 10
   <211> 31
   <212> DNA
   <213> Artificial
<220>
   <223> Genotyping Primers
<400> 10
   gcgggcaggg cggcttgtgc agccctaaag g 31
<210> 11
   <211> 23
   <212> DNA
   <213> Artificial
<220>
   <223> Genotyping Primers
<400> 11
   gcgggcttgt gcagccctaa aga 23
<210> 12
   <211> 21
   <212> DNA
   <213> Artificial
<220>
   <223> Genotyping Primers
<400> 12
   caggaaaggc cattgtggag a 21
<210> 13
   <211> 32
   <212> DNA
   <213> Artificial
<220>
   <223> Genotyping Primers
<400> 13
   gcgggcaggg cggcgaagac cagccagtgc ac 32
<210> 14
   <211> 24
   <212> DNA
   <213> Artificial
<220>
   <223> Genotyping Primers
<400> 14
   gcgggcgaag accagccagt gcat 24
<210> 15
   <211> 21
   <212> DNA
   <213> Artificial
<220>
   <223> Genotyping Primers
<400> 15
   caaggctttg ccctcagggt t 21
<210> 16
   <211> 28
   <212> DNA
   <213> Artificial
<220>
   <223> Genotyping Primers
<400> 16
   gcgggcaggg tgcacctttt ggacattc 28
<210> 17
   <211> 21
   <212> DNA
   <213> Artificial
<220>
   <223> Genotyping Primers
<400> 17
   gcgtgcacct tttggacatt t 21
<210> 18
   <211> 18
   <212> DNA
   <213> Artificial
<220>
   <223> Genotyping Primers
<400> 18
   gaacgcacgt cccttgtc 18
<210> 19
   <211> 30
   <212> DNA
   <213> Artificial
<220>
   <223> Genotyping Primers
<400> 19
   gcgggcaggg ccgtgtttga ctcaactcag 30
<210> 20
   <211> 23
   <212> DNA
   <213> Artificial
<220>
   <223> Genotyping Primers
<400> 20
   gcgccgtgtt tgactcaact caa 23
<210> 21
   <211> 20
   <212> DNA
   <213> Artificial
<220>
   <223> Genotyping Primers
<400> 21
   tttgccggaa atattagcgt 20
<210> 22
   <211> 27
   <212> DNA
   <213> Artificial
<220>
   <223> Genotyping Primers
<400> 22
   gcgggcaggg ttgtgcagcc ctaaagg 27
<210> 23
   <211> 20
   <212> DNA
   <213> Artificial
<220>
   <223> Genotyping Primers
<400> 23
   gcgttgtgca gccctaaaga 20
<210> 24
   <211> 21
   <212> DNA
   <213> Artificial
<220>
   <223> Genotyping Primers
<400> 24
   caggaaaggc cattgtggag a 21
<210> 25
   <211> 28
   <212> DNA
   <213> Artificial
<220>
   <223> Genotyping Primers
<400> 25
   gcgggcaggg gaagaccagc cagtgcac 28
<210> 26
   <211> 21
   <212> DNA
   <213> Artificial
<220>
   <223> Genotyping Primers
<400> 26
   gcggaagacc agccagtgca t 21
<210> 27
   <211> 21
   <212> DNA
   <213> Artificial
<220>
   <223> Genotyping Primers
<400> 27
   caaggctttg ccctcagggt t 21
<210> 28
   <211> 44
   <212> DNA
   <213> Artificial
<220>
   <223> Genotyping Primers
<400> 28
   gcgggcaggg cggcgggggc ggggcctgca ccttttggac attc 44
<210> 29
   <211> 36
   <212> DNA
   <213> Artificial
<220>
   <223> Genotyping Primers
<400> 29
   gcgggcaggg cggcggggtg caccttttgg acattt 36
<210> 30
   <211> 18
   <212> DNA
   <213> Artificial
<220>
   <223> Genotyping Primers
<400> 30
   gaacgcacgt cccttgtc 18
<210> 31
   <211> 46
   <212> DNA
   <213> Artificial
<220>
   <223> Genotyping Sequence
<400> 31
   gcgggcaggg cggcgggggc ggggccccgt gtttgactca actcag 46
<210> 32
   <211> 38
   <212> DNA
   <213> Artificial
<220>
   <223> Genotyping Primers
<400> 32
   gcgggcaggg cggcggggcc gtgtttgact caactcaa 38
<210> 33
   <211> 20
   <212> DNA
   <213> Artificial
<220>
   <223> Genotyping Primers
<400> 33
   tttgccggaa atattagcgt 20
<210> 34
   <211> 43
   <212> DNA
   <213> Artificial
<220>
   <223> Genotyping Primers
<400> 34
   gcgggcaggg cggcgggggc ggggccttgt gcagccctaa agg 43
<210> 35
   <211> 35
   <212> DNA
   <213> Artificial
<220>
   <223> Genotyping Primers
<400> 35
   gcgggcaggg cggcggggtt gtgcagccct aaaga 35
<210> 36
   <211> 21
   <212> DNA
   <213> Artificial
<220>
   <223> Genotyping Primers
<400> 36
   caggaaaggc cattgtggag a 21
<210> 37
   <211> 44
   <212> DNA
   <213> Artificial
<220>
   <223> Genotyping Primers
<400> 37
   gcgggcaggg cggcgggggc ggggccgaag accagccagt gcac 44
<210> 38
   <211> 36
   <212> DNA
   <213> Artificial
<220>
   <223> Genotyping Primers
<400> 38
   gcgggcaggg cggcggggga agaccagcca gtgcat 36
<210> 39
<211> 21
   <212> DNA
   <213> Artificial
<220>
   <223> Genotyping Primers
<400> 39
   caaggctttg ccctcagggt t 21
<210> 40
   <211> 33
   <212> DNA
   <213> Artificial
<220>
   <223> Genotyping Primers
<400> 40
   gcgggcaggg cggctgcacc ttttggacat tct 33
<210> 41
   <211> 25
   <212> DNA
   <213> Artificial
<220>
   <223> Genotyping Primers
<400> 41
   gcgggctgca ccttttggac atttt 25
<210> 42
   <211> 17
   <212> DNA
   <213> Artificial
<220>
   <223> Genotyping Primers
<400> 42
   gaacgcacgt cccttgt 17
<210> 43
   <211> 35
   <212> DNA
   <213> Artificial
<220>
   <223> Genotyping Primers
<400> 43
   gcgggcaggg cggcccgtgt ttgactcaac tcagc 35
<210> 44
   <211> 27
   <212> DNA
   <213> Artificial
<220>
   <223> Genotyping Primers
<400> 44
   gcgggcccgt gtttgactca actcaac 27
<210> 45
   <211> 20
   <212> DNA
   <213> Artificial
<220>
   <223> Genotyping Primers
<400> 45
   tttgccggaa atattagcgt 20
<210> 46
   <211> 32
   <212> DNA
   <213> Artificial
<220>
   <223> Genotyping Primers
<400> 46
   gcgggcaggg cggcttgtgc agccctaaag ga 32
<210> 47
   <211> 24
   <212> DNA
   <213> Artificial
<220>
   <223> Genotyping Primers
<400> 47
   gcgggcttgt gcagccctaa agaa 24
<210> 48
   <211> 21
   <212> DNA
   <213> Artificial
<220>
   <223> Genotyping Primers
<400> 48
   caggaaaggc cattgtggag a 21
<210> 49
   <211> 33
   <212> DNA
   <213> Artificial
<220>
   <223> Genotyping Primers
<400> 49
   gcgggcaggg cggcgaagac cagccagtgc acc 33
<210> 50
   <211> 25
   <212> DNA
   <213> Artificial
<220>
   <223> Genotyping Primers
<400> 50
   gcgggcgaag accagccagt gcatc 25
<210> 51
   <211> 21
   <212> DNA
   <213> Artificial
<220>
   <223> Genotyping Primers
<400> 51
   caaggctttg ccctcagggt t 21
<210> 52
   <211> 29
   <212> DNA
   <213> Artificial
<220>
   <223> Genotyping Primers
<400> 52
   gcgggcaggg cggcgggtct gaggctgcg 29
<210> 53
   <211> 18
   <212> DNA
   <213> Artificial
<220>
   <223> Genotyping Primers
<400> 53
   gcggggtctg aggctgcc 18
<210> 54
   <211> 18
   <212> DNA
   <213> Artificial
<220>
   <223> Genotyping Primers
<400> 54
   aggccgttgg agccgaac 18